Europäisches Patentamt

European Patent Office

Office européen des brevets

(11) Publication number: **0 517 925 A1**

## EUROPEAN PATENT APPLICATION
### published in accordance with Art. 158(3) EPC

(21) Application number: **92901913.1**

(22) Date of filing: **27.12.91**

(86) International application number:
**PCT/JP91/01803**

(87) International publication number:
**WO 92/12177 (23.07.92 92/19)**

(51) Int. Cl.⁵: **C07K 15/04**, C12P 21/00,
A61K 37/02, //C12N15/19,
(C12P21/00,C12R1:91)

(30) Priority: **28.12.90 JP 415440/90**

(43) Date of publication of application:
**16.12.92 Bulletin 92/51**

(84) Designated Contracting States:
**AT BE CH DE ES FR GB IT LI LU NL SE**

(71) Applicant: **ASAHI KASEI KOGYO KABUSHIKI KAISHA**
**2-6, Dojimahama 1-chome Kita-ku**
**Osaka-shi, Osaka 530(JP)**

(72) Inventor: **KONDO, Shuhei 139-1-607,**
**Nishishiiji**
**Numazu-shi**
**Shizuoka-ken 410-03(JP)**
Inventor: **OGAWA, Kohei B23, Endo**
**Apartment**
**68, Aoshima-cho**
**Fuji-shi Shizuoka-ken 417(JP)**

(74) Representative: **Smulders, Theodorus A.H.J., Ir.**
**Vereenigde Octrooibureaux Nieuwe Parklaan 97**
**NL-2587 BN 's-Gravenhage(NL)**

(54) **NOVEL MEGAKARYOCYTE AMPLIFIER AND PRODUCTION THEREOF.**

㊼ A substantially pure, novel megakaryocyte amplifier protein which has a molecular weight of 25,000 ± 8,000 as determined by gel filtration and an isoelectric point of 8 ± 1 as determined by isoelectric electrophoresis, can be discriminated from human erythropoietin, interleukin 1α, interleukin 1β, interleukin 6 and interleukin 7, does not have any megakaryocyte colony stimulating factor activity, and can activate megakaryocyte amplification. This protein can be produced by the cell culture or genetic engineering technologies. Since it has activities of promoting megakaryocyte amplification and increasing platelets in the peripheral blood, it is effective in preventing and treating thrombocytopenia.

Rank Xerox (UK) Business Services

# FIG. 4 — (a)

**AchE activity(serum-free)**

IL-3(μl)

Legend:
- ■ Control
- ▨ IL-6:100ng/ml
- ▦ Present invention: 0.1μl
- ▨ Present invention: 1.0μl
- □ Present invention: 10.0μl

Technical Field

The present invention relates to a novel megakaryocyte potentiator and a method for producing the same. More particularly, the present invention is concerned with a novel megakaryocyte potentiator protein having the activity to promote the proliferation and differentiation of a megakaryocyte which is a platelet precursor cell, thereby promoting the production of platelets. The present invention is also concerned with a method for producing the megakaryocyte potentiator protein by cell culturing or genetic engineering technique. The present invention is also concerned with a pharmaceutical composition comprising the above novel megakaryocyte potentiator protein, which is useful for the prevention and treatment of diseases, such as thrombocytopenia and the like.

Background Art

Platelets play an important role in promoting the thrombus formation and blood coagulation which serve to spontaneously stop bleeding caused by the break of a blood vessel in a living body. In the past more than 20 years, many researchers have been making efforts to obtain thrombopoietin (TPO), which is considered to be a factor specifically promoting the production of platelets. However, no conventional attempts has succeeded in obtaining TPO.

From the fact that when the plasma of an animal having thrombocytopenia is injected into a normal animal, the production of platelets in the normal animal is increased, whereas when the platelets is injected into a animal, the production of platelets therein is lowered, the existence of TPO having the activity to regulate the production of platelets in accordance with the increase and the decrease in the number of platelets has long been proposed. From the results of studies made after the proposal, it has been made clear that in bone marrow a megakaryocyte matures from a megakaryocyte precursor cell which has differentiated from a bone marrow stem cell and then, platelets are shed from the matured megakaryocyte into blood. From the results of in vitro experiments, it has also been made clear that megakaryocyte colony stimulating factor (Meg-CSF) functions in the early stage of the process of proliferation and differentiation of a megakaryocyte, and that TPO has the activity to promote the maturation of a megakaryocyte functions in the later stage of the process of proliferation and differentiation of a megakaryocyte. Illustratively stated, a megakaryocyte precursor cell repeats cell division when stimulated by Meg-CSF to thereby increase the number of megakaryocyte compartments. Then each of the megakaryocyte precursor cells undergoes endomitosis by the action of TPO, so that the ploidy of the chromosome is increased (up to 32N) and, at the same time, the cytoplasm matures and grows in size, to thereby produce platelets. TPO is also called megakaryocyte potentiator (Meg-POT).

The activity of Meg-CSF is determined by measuring the activity to form megakaryocyte colonies in in vitro soft agar culturing of human or mouse bone marrow cells. At present, the activity of Meg-CSF is found, for example, in the urines of a patient having aplastic anemia and of a patient having sudden thrombocytopenic purpura, in the plasma of a patient having megakaryocyte-aplastic thrombocytopenia, in the supernatant of a culture of human leucocytes having been stimulated by kidney bean lectin, and in the supernatant of a culture of leukemic mouse cell WEHI-3 line.

It has become clear that of various cytokines, interleukin 3 (IL-3) (hereinafter, interleukin is referred to simply as "IL") is a Multi-CSF capable of acting nonspecifically on many systems including megakaryocytepoiesis system. Further, based on various findings, such as the finding that the Meg-CSF in the supernatant of a culture of WEHI-3 line is completely identical with IL-3, it is believed that most of the Meg-CSF activities which have conventionally been found in the supernatants of cell cultures are ascribed to IL-3. However, an Meg-CSF which acts specifically on platelet-related cells, has not yet been known.

On the other hand, the activity of TPO is determined by measuring the activity to enhance the colony forming activity of Meg-CSF and/or the activity to promote the maturation of a megakaryocyte. Heretofore, attempts have been made to provide several factors having TPO-like activities. For example, megakaryocyte stimulatory factor (MSF) and a method for producing the same have been reported, in which MSF is prepared from the supernatant of a culture of a human fetal kidney cell line and has a molecular weight of 15,000 as measured by SDS-PAGE and an isoelectric point of 5.1 and has an activity of promoting protein synthesis in a megakaryocyte (see U.S. Patent No. 4,894,440). Further, it has recently been confirmed that IL-6 (which was found as a glycoprotein inducing the antibody production of a B cell and is a multifunctional cytokine known to participate in immune systems and acutephase response systems and act on malignant tumor) also participates in hematopoietic systems, so that it exhibits in vitro an Meg-POT activity and the activity to promote the maturation of a megakaryocyte [Ishibashi, T. et al., Proc. Natl. Acad. Sci. USA, 86, 5953 (1989)], and exhibits in vivo the activity to promote the production of platelets [Asano, S. et al., Blood, 75, 1602 (1990)]. Further, it has been reported that IL-7, IL-11 and the like also have a megakaryocyte potentiator activity. However, the megakaryocyte potentiator activities of these factors are weak, and whether or not these factors are hematopoietic factors which are innate and constitutive in the living body, is unclear.

In the above context of the art, the present inventors made extensive and intensive studies with a view toward developing a novel megakaryocyte potentiator having the activity to specifically and strongly promote the proliferation and differentiation of a megakaryocyte and the activity to promote the production of platelets. As a result, the present inventors have unexpectedly found a novel megakaryocyte potentiator in the supernatant of an animal cell culture and have also found that by adding an appropriate promoting agent to the culture medium, the megakaryocyte potentiator can be produced in a large quantity. Further, the present inventors not only isolated and purified the megakaryocyte potentiator from the supernatant of the culture, to thereby characterize the megakaryocyte potentiator, but also demonstrated the utility thereof as a pharmaceutical. Moreover, the megakaryocyte potentiator can be produced by gene expression using genetic engineering technique. On the basis of these findings, the present invention has been completed.

Disclosure of the Invention

Accordingly, it is an object of the present invention to provide a substantially pure, novel megakaryocyte potentiator having the strong activity to promote the proliferation and differentiation of a megakaryocyte.

It is another object of the present invention to provide a method for producing a megakaryocyte potentiator, which comprises culturing animal cells in a culture medium to thereby produce a megakaryocyte potentiator protein in a conditioned medium, collecting a supernatant from the conditioned medium, and isolating and purifying the megakaryocyte potentiator protein from the supernatant, thereby obtaining a megakaryocyte potentiator protein having the activity to promote the proliferation and differentiation of a megakaryocyte.

It is still another object of the present invention to provide a method for producing a megakaryocyte potentiator, which is in accordance with the above-mentioned method and wherein the culture medium for culturing the animal cells contains an agent capable of promoting the production of a megakaryocyte potentiator protein, thereby causing a megakaryocyte potentiator to be produced in an increased quantity.

It is a further object of the present invention to provide a method for producing a megakaryocyte potentiator by genetic engineering technique.

It is still a further object of the present invention to provide a pharmaceutical composition containing a therapeutically effective amount of a megakaryocyte potentiator protein as an active ingredient, and a method for treatment using the pharmaceutical composition.

The foregoing and other objects, features and advantages of the present invention will be apparent to those skilled in the art from the following detailed description and appended claims taken in connection with the accompanying drawings.

According to the present invention, there is provided a substantially pure megakaryocyte potentiator protein having the activity to promote the proliferation and differentiation of a megakaryocyte and which has the following characteristics:

(a) a molecular weight of 25,000 ± 8,000 as measured by a gel filtration method;

(b) an isoelectric point of 8 ± 1 as measured by an isoelectric focusing method;

(c) the activity to promote the proliferation and differentiation of a megakaryocyte is substantially not lowered when the protein is tested after exposure to each of the antibodies against human erythropoietin, human interleukin $1\alpha$, human interleukin $1\beta$, human interleukin 6 and human interleukin 7; and

(d) no megakaryocyte colony stimulating factor activity.

The above-mentioned antibodies against human erythropoietin, human interleukin $1\alpha$, human interleukin $1\beta$, human interleukin 6 and human interleukin 7, are available from Genzyme CO., the U.S.A. (the 1991 Genzyme Catalog; Cytokine Research Products) and can be used in the present invention.

In another aspect of the present invention, there is provided a method for producing a megakaryocyte potentiator, which method comprises culturing animal cells in a culture medium to thereby produce a megakaryocyte potentiator protein in a conditioned medium, collecting a supernatant from the conditioned medium, and isolating and purifying the megakaryocyte potentiator protein from the supernatant, thereby obtaining a megakaryocyte potentiator protein having the activity to promote the proliferation and differentiation of a megakaryocyte.

The animal cell to be used in the method of the present invention can be various types of cells as long as the cells are capable of producing a megakaryocyte potentiator having not only the activity to promote the proliferation and differentiation of a megakaryocyte but also the activity to increase the production of platelets in peripheral blood. Normal diploid cells can be advantageously employed. For example, human cells derived from kidney, intestines, lung, heart, ureter, skin, foreskin, tongue, thyroid gland, placenta or uterus, preferably human cells derived from fetal kidney, fetal lung or fetal foreskin, more preferably human cells derived from fetal lung can be used.

The megakaryocyte potentiator protein can also be isolated and purified from an extraction of the above-mentioned tissues. However, more preferably, the megakaryocyte potentiator protein can be obtained by a method which comprises culturing cells derived from one of the above-mentioned tissues in an appropriate growth medium to thereby produce a megakaryocyte potentiator protein in a conditioned medium, collecting a supernatant from the conditioned medium, and isolating and purifying the megakaryocyte potentiator protein from the collected supernatant. It is preferred that growth of these cells be conducted according to a culturing method which is generally used in cell culturing, for example, a method described in "Soshiki Baiyo (Tissue Culture)" (edited by Jun-nosuke Nakai et al., published in 1976 by Asakura Shoten). These cells are cultured in a culture medium containing a carbon source and nitrogen source and, if desired, inorganic salts and/or other additives, to thereby produce a megakaryocyte potentiator protein. Further, according to the present invention, the production of the megakaryocyte potentiator protein in the conditioned medium can be dramatically increased by culturing the cells in a culture medium containing an agent capable of promoting the production of the megakaryocyte potentiator protein, preferably a peptone obtained by enzymatic digestion of aminal meat. With respect to the concentration of the peptone obtained by enzymatic digestion of aminal meat, peptone can be used in 0 to 4 w/v%, preferably 0.1 to 2 w/v%. The peptone obtained by enzymatic digestion of animal meat is generally used in culture media for bacteria and is usually called proteose peptone or animal-meat peptone.

The method for preparing the peptone obtained by enzymatic digestion of animal meat is conventional. For example, the method described in "Saikin Baichigaku Koza Dainishu (The Study of Culture Medium for Bacteria, the Second Series)" (written by Toshikazu Sakazaki, published by Naya Shoten, 1967) can be used. As animal meat, the meat or internal organs of cattle, pigs, fowl, sheep, whales and the like can be used. Of these, the meat of cattle is most preferred. As an enzyme for digestion, trypsin, papain, pepsin, pancreatin and the like can be used. Meat as mentioned above is minced and mixed with water. The pH value of the obtained mixture is adjusted with sodium carbonate, concentrated hydrochloric acid and the like to an appropriate pH value for enzymatic digestion. An enzyme is added to the mixture and then, incubated at 20 to 40 °C for 1 to 20 days, usually at 37 °C for 2 to 3 days, to thereby perform enzymatic digestion. After the digestion, the mixture is heated at 100 °C or more in order to inactivate the enzyme and to perform thermocoagulation of undigested protein and, the inactivated enzyme and the coagulated protein are then removed by filtration. The resultant filtrate is concentrated, dried and pulverized. As a method for the concentration, drying and pulverization, either a method in which the filtrate is boiled down, followed by pulverization, or a method in which the filtrate is lyophilized, followed by pulverization, can be employed. Commercially available peptones include Proteose Peptone No. 1, Proteose Peptone No. 2, Proteose Peptone No. 3 and Thiopepton (all of which are manufactured and sold by Difco Laboratories, U.S.A.), Proteose Peptone L46 and Peptone PL46 (both of which are manufactured and sold by Oxoid Limited, England), Thioton (manufactured and sold by British Bio-Technology Ltd., England), Proteose Peptone (manufactured and sold by Daigo Eiyo Kagaku, Co., Ltd., Japan) and the like.

An example of the production of the megakaryocyte potentiator by the cell culturing method is described below. Cells obtained by primary cell culture of cells taken from tissue and capable of producing a megakaryocyte potentiator, or commercially available cells capable of producing megakaryocyte potentiator, are cultured by adhesion culturing or suspension culturing. Illustratively stated, the cells are seeded to the culture medium at an appropriate density, preferably at a density of $10^5$ cells/ml together with 0.1 to 10 mg/ml of carrier beads for cell culturing and then, cultured in the presence of serum at 15 to 45 °C, preferably 25 to 45 °C, at a pH value of the culture medium in the range of 5 to 9, preferably 6 to 8, in air containing 5 % $CO_2$. When an agent for promoting the production of the megakaryocyte potentiator is used, the culturing for producing a megakaryocyte potentiator is performed under serum-free conditions, in a concentration of the agent of 0 to 4 %, preferably 0.1 to 2 %. The culturing is performed preferably after satisfactory growth of the cells has been attained, more preferably after the cells are in a confluent condition. The number of days for the culturing is generally 1 to 60 days but may exceed 60 days. Since the rate of the production of megakaryocyte potentiator becomes gradually lower in the late phase of the production, in the case of commercial production, the number of days for the culturing is selected so that the efficiency of the production is highest. Under the above-mentioned conditions, the megakaryocyte potentiator is produced in and shed from the cells in the culture. The quantity of the megakaryocyte potentiator produced can be measured by the method for measuring the the megakaryocyte potentiator activity, which is described in Reference Examples 1(a) and 1(b). The maturation degree of the megakaryocyte potentiator can be confirmed by the method for measuring the DNA content of the megakaryocyte potentiator, which is described in Reference Example 2.

In another aspect of the present invention, there is provided a method for producing a megakaryocyte potentiator, which comprises expressing a megakaryocyte potentiator gene in an appropriate host cell by conventional genetic engineering technique, and collecting and purifying the expressed megakaryocyte potentiator.

Illustratively stated, total RNA is extracted from a cell, which is capable of producing a megakaryocyte potentiator having not only the activity to promote the proliferation and differentiation of the megakaryocyte but also the activity to increase the number of platelets in peripheral blood. Examples of such cells include human cells derived from the kidney, intestines, lung, heart, ureter, skin, foreskin, tongue, thyroid gland, placenta and uterus; and preferably include human cells derived from fetal kidney, lung and foreskin, more preferably human cells derived from fetal lung. From the extracted total RNA, poly $A^+$ RNA is obtained by purification. Using an appropriate expression vector (preferably an expression vector for an eukaryote), the poly $A^+$ RNA and a linker, a cDNA library is prepared. Appropriate host cell (first host cells), for example, E. coli is transformed with the cDNA library. Using a culture of the resultant transformant, a plasmid DNA is prepared. Appropriate host cells (second host cells), preferably cells derived from an animal, more preferably the COS cells derived from monkey, is transfected with the above-obtained plasmid DNA, and the transfected COS cells are cultured and caused to express the megekaryocyte potentiator gene. By collecting and purifying the expression product, the megakaryocyte potentiator can be obtained.

An example of the above procedure is illustrated below. The total RNA is extracted from an appropriate amount of cells capable of producing a megakaryocyte potentiator, for example, from $10^8$ cells of human fetal lung cells, using an RNA isolation kit (for example, a kit manufactured and sold by Invitrogen CORPORATION, U.S.A,; catalog No. K1592-01) by the guanidine isothiocyanate method according to the manual attached thereto. Then, a poly $A^+$ RNA is obtained therefrom according to the conventional method. The conventional method of Oligodex-dT30 (manufactured and sold by Japan Synthetic Rubber Co., Ltd, Japan) may be used. By the above method, generally, about 200 $\mu$g of total RNA and 1 to 2 $\mu$g of poly $A^+$ RNA are obtained. Subsequently, according to the Okayama-Berg method, a cDNA library is prepared, in which, for example, a 3'-oligo(dT)-tailed pcDV-1 expression vector for eukaryote (manufactured and sold by Pharmacia Fine Chemicals AB, Sweden, No. 27-4955-01), the above-obtained poly $A^+$ RNA and a 3'-oligo-(dG)-tailed pL1 linker (manufactured and sold by Pharmacia Fine Chemicals AB, Sweden, No. 27-4957) can be used. Alternatively, pcDL-SR$\alpha$296 may be used. The obtained solution containing a cDNA library is divided into an appropriate number of pools, preferably 10 to 200 pools, more preferably 50 to 100 pools. Using the pools individually, E. coli MC1061 strain (ATCC53338) is transformed. The transformed E. coli is cultured overnight in the presence of ampicillin. After collection and bacteriolysis of the cells, a plasmid DNA is prepared using, for example, Quiagen-tip-100 (manufactured and sold by Quiagen, U.S.A.), according to the manual attached thereto. The obtained recombinant DNA is transfected into an appropriate host cell, preferably simian kidney cell COS1 (ATCC, CRL1650) by the diethylaminoethyl-dextran method (CURRENT PROTOCOLS IN MOLECULAR BIOLOGY 9.2.1-9.2.6). Then, gene expression is conducted in the same manner as described in Example 2 in the specification of International Patent Application No. WO88/05053. Illustratively stated, the transformed cells are cultured under appropriate culturing conditions, for example, conditions such that culturing is conducted in D-MEM culture medium (manufactured and sold by Flow Laboratories, Inc., U.S.A.) containing 10 % fetal calf serum at 37 °C for 40 hours in an atmosphere containing 5% $CO_2$. After culturing, the culture medium is replaced with fresh D-MEM culture medium containing no serum and then further culturing is conducted. During the further culturing, the culture is collected three times at intervals of two days.

Further, according to the present invention, the expression cells containing the megakaryocyte potentiator gene, may be screened using the megakaryocyte potentiator activity as a criterion, to thereby clone the megakaryocyte potentiator gene.

Illustratively stated, each of the collected cultures is concentrated and subjected to determination of the megakaryocyte potentiator activity, e.g., by measuring acetylcholinesterase activity through liquid culturing. Using the megakaryocyte potentiator activity as a criterion, the pools containing megakaryocyte potentiator gene can be selected. In this instance, a secondary screening of the cDNA library may be conducted. That is, with the above-obtained DNAs positive with respect to the desired gene, E. coli is transformed to thereby obtain colonies. The obtained colonies (about 2,000 colonies) are separated into groups each consisting of about 10 colonies, and cultured. Then, preparation of DNAs, transfection into COS1 cells, expression of the gene and measurement of the acetylcholinesterase activity are conducted in the same manner as mentioned above, to thereby perform a secondary screening of the cDNA library. In general, by performing the screening several times according to the above method, E. coli containing a cDNA plasmid capable of expressing a megakaryocyte potentiator is isolated [Hayashida, K. et al. "Hematopoietic Factor" 1, No. 2, 102-108(1990)].

Further, according to the present invention, a genetic probe coding for a part of the primary structure of the megakaryocyte potentiator protein is prepared, and the megakaryocyte potentiator gene can be cloned using the prepared genetic probe.

Still further, according to the present invention, host cells, such as E. coli, yeast, simian kidney cells (COS cell), Chinese hamster ovarian cells (CHO cell), mouse C127 cells, a human fetal kidney cell line, silkworm SF9 cells and the like, are transfected with the cloned megakaryocyte potentiator gene, so that a megakaryocyte potentiator can be efficiently produced by the transfected cells according to the expression of the gene. The produced megakaryocyte potentiator is collected and purified, to thereby obtain the megakaryocyte potentiator.

In the method of the present invention for producing the megakaryocyte potentiator, when the production is performed by cell culturing, a supernatant of the culture is collected at the time when the quantity of the megakaryocyte potentiator produced has reached a desired level or a predetermined number of days has passed from the start of the culturing. As a method for isolating and purifying the megakaryocyte potentiator, the conventional methods which have been used in protein chemistry, for example, adsorption by means of a carrier, salting out, electrophoresis, and various types of chromatographies in which ion exchange, gel filtration and affinity for an appropriate ligand are respectively utilized, can be employed individually or in combination. Preferred examples of chromatographies include CM sepharose column chromatography using a sepharose having bonded thereto a carboxymethyl group, gel-filtration column chromatography using particles of, e.g., crosslinked dextran gel, dye-adsorption column chromatography, and antibody-affinity column chromatography using an antibody specific for the megakaryocyte potentiator of the present invention.

The thus obtained novel megakaryocyte potentiator has not only the activity to promote the proliferation and differentiation of a megakaryocyte but also the activity to increase the number of platelets in peripheral blood. The megakaryocyte potentiator can be used as a reagent for studies on the differentiation, multiplication and maturation of a megakaryocyte from bone marrow stem cells and bone marrow megakaryocyte precursor cells. Further, the megakaryocyte potentiator can also be used as a pharmaceutical either in a single form or in a composition form in which a therapeutically effective amount of the megakaryocyte potentiator is added to at least one pharmaceutically acceptable carrier, diluent or excipient to provide an appropriate dosage form. As a carrier, diluent and excipient, the conventional carrier, diluent and excipient can be used. Furthermore, the megakaryocyte potentiator may be blended with at least one factor selected from the group consisting of IL-1, IL-2, IL-3, IL-4, IL-5, IL-6, IL-7, IL-8, IL-9, IL-11, GM-CSF, G-CSF, M-CSF, SCF, IFNs, LIF, TNF and EPO, and at least one pharmaceutically acceptable carrier, diluent or excipient, to provide an appropriate dosage form suitable for use as a pharmaceutical.

The megakaryocyte potentiator of the present invention can be used for the treatment and/or prevention of certain types of thrombocytopenia, for example, various types of thrombocytopenia caused by administration of an anticancer agent, radiotherapy, deficiency of the megakaryocyte potentiator, aplastic anemia, implantation of bone marrow and autoimmune disease. The megakaryocyte potentiator of the present invention can also be used for treating leukemia. Further, the megakaryocyte potentiator of the present invention can be used as a substitute or adjuvant for a platelet transfusion, and can be used for in vitro multiplication, by culturing, of bone marrow cells for transfusion.

The megakaryocyte potentiator of the present invention can also be used as an injection. In this case, a thickening agent such as sucrose, glycerin, methyl cellulose, carboxymethyl cellulose and the like, and a pH adjustor such as various inorganic salts, can be used as additives for the injection.

The dose of the megakaryocyte potentiator of the present invention varies depending on the age, sex, weight, conditions, etc. of the patient. In general, however, the dose is 0.1 $\mu$g to 100 mg. The megakaryocyte potentiator may be administered once a day or, if desired, several times a day.

Best Mode for Carrying Out the Invention

Hereinbelow, the present invention will be illustrated with reference to Examples, which however should not be construed as limiting the scope of the present invention.

Reference Example 1 [the method for the measurement of the activity to promote the proliferation and differentiation of a megakaryocyte]

The activity of the novel megakaryocyte potentiator protein of the present invention was measured according to the following methods (a) and (b).

(a) Measurement of Meg-POT activity by soft agar culturing:

Preparation of a bone marrow cell suspension

The IMDM (Isocove's modification of Dallbecco's medium) solution to be used in the subsequent procedure for preparing a suspension of bone marrow cells, was prepared as follows. 3.024 g of sodium bicarbonate and 3.04 $\mu$l of $\beta$-mercaptoethanol were added to IMDM powder (in an amount for a final liquid volume of 1 liter) (manufactured and sold by GIBCO, U.S.A.). The pH value of the resultant mixture was adjusted to 7.1 and then diluted in a messcylinder so that the total volume of the mixture became 1 liter. To the resultant mixture were further added 50 IU/ml of penicillin and 50 $\mu$g/ml streptomycin (both of which are manufactured and sold by Flow Laboratories, Inc., U.S.A.), to thereby obtain an IMDM solution.

A thighbone was taken from C57BL/6 male mouse (6-9 weeks old). The upper portion of the thighbone was cut off. Then, a 10 ml plastic syringe (with a 22G needle) containing 10 ml of the above-prepared IMDM solution, was pushed, at its needle, into the knee joint of the thighbone and then, the contents of the syringe were injected, so that the bone marrow was vigorously pushed out from the upper, cut-off end of the thighbone onto a plastic dish having a diameter of 10 mm. The bone marrow cells were separated from each other by an 8-time pipetting operation of a syringe (6 times with a 19G needle and 2 times with a 22G needle). The bone marrow cells were transferred into a Falcon tube having a volume of 15 ml, and the unprecipitated cells were collected. The collected cells were washed twice with 10 ml of the IMDM solution. The cells were then suspended into the IMDM solution in a concentration such that the cells taken from one mouse are contained in 2.5 ml of the IMDM solution, and well dispersed, to thereby obtain a suspension of the bone marrow cells to be used for the subsequent colony assay. The cell concentration of the suspension was measured by means of a hemocytometer by Trypan Blue (manufactured and sold by Flow Laboratories, Inc., U.S.A.) staining method.

Colony assay

As an acetylcholinesterase staining solution, a solution in which acetylcholinesterase was dissolved into 400 ml of 75 mM phosphate buffer (pH 6.0) containing 1.73 mM acetylthiocholine iodide, 0.5 mM potassium ferricyanide, 5 mM sodium citrate and 3 mM copper sulfate (all of them are manufactured and sold by Wako Pure Chemical Industries, Ltd., Japan), was used in the subsequent procedure.

Horse serum (manufactured and sold by J.R. Scientific, INC., U.S.A.) was added to a bone marrow cell-in-IMDM suspension containing $10^5$ of the bone marrow cells in a plastic dish having a diameter of 100 mm (the amount of the horse serum was such that the final concentration of the horse serum in the below-mentioned IMDM culture medium became 15 v/v%) and then, added thereto were 50 ng of IL-3 (manufactured and sold by Genzyme Corporation, U.S.A.) or the supernatant of a COS1 cell culture containing IL-3 (the amount of the supernatant was such that the final concentration of the supernatant became 1 v/v%) and 0.3 % Bactoagar (manufactured and sold by DIFCO, U.S.A.), to thereby obtain 500 $\mu$l of an IMDM culture medium in a soft agar state. A sample solution to be measured with respect to the Meg-POT activity thereof was added to the thus obtained IMDM culture medium and then, culturing was conducted at 37 °C for 7 days in an atmosphere of 5 % $CO_2$. The preparation of the above-mentioned supernatant of the COS1 cell culture containing IL-3, had been done by transforming COS1 cells (ATCC CRK1650) with a plasmid DNA in which mouse IL-3 cDNA was linked with SV-40 promoter, expressing the IL-3 cDNA in substantially the same manner as described in Example 2 of WO88/05053, and obtaining a supernatant from the culture containing IL-3.

When the culturing of the bone marrow cells was conducted under serum-free conditions, the culturing was performed in substantially the same manner as mentioned above except that in place of the above-mentioned IMDM culture medium containing 15 v/v% horse serum, use was made of 500 $\mu$l of an IMDM culture medium containing 1 w/v% bovine serum albumin, 360 $\mu$g/ml human transferrin, 0.98 $\mu$g/ml cholesterol and 50 ng of IL-3 or the supernatant of a culture of COS1 cells containing 1 v/v% IL-3. After completion of the culturing, the agar disk was transferred onto a slide glass and dried. The dried agar disk was fixed with 2 % glutaraldehyde. The fixed agar disk was washed with a phosphate-buffered saline (PBS), followed by staining the megakaryocyte specifically with the above-mentioned acetylcholinesterase staining solution. The number of colonies was counted using AHB type microscope (manufactured and sold by Olympus Optical Co., Ltd.), wherein any colony comprised of at least 6 positive cells was counted as one

8

colony.

(b) Measurement of acetylcholinesterase activity by liquid culturing:

Diisopropyl fluorophosphate (DFP)(manufactured and sold by Sigma Chemical Company, U.S.A.) was added to a suspension of mouse bone marrow cells in a final concentration of 0.4 mM, which suspension had been prepared in substantially the same manner as described in item (a) above. The resultant mixture was allowed to stand at room temperature for 20 minutes while occasionally stirring, to thereby inactivate the inherent acetylcholinesterase activity of the bone marrow cells. The cell number was counted by means of a hemocytometer in the same manner as mentioned above.

The suspension of the bone marrow cells was suspended into an IPDM solution at a density of the bone marrow cells of $2.5 \times 10^5$ to $5 \times 10^5$ cells/ml, which IPDM solution contained 15 v/v% horse serum in which the inherent acetylcholinesterase activity had been inactivated by DFP (J. Cell. Physiol., $\underline{122}$, 159, 1985). The resultant suspension was placed in a 96-well dish for culturing (manufactured and sold by Sumitomo Bakelite Co., Ltd., Japan) in an amount of 0.2 ml per well. 20 $\mu$l of the sample solution was added to each well, followed by culturing at 37 °C for 7 days in an atmosphere of 5 % $CO_2$.

Then, centrifugation was conducted to precipitate the cells, and then the supernatant was removed. To each well were added 20 $\mu$l of 5.6 mM acetylthiocholine iodide (manufactured and sold by Sigma Chemical Company, U.S.A.), 180 $\mu$l of 0.12 M NaCl, 1 mM EDTA and 50 mM Hepes buffer (pH 7.5) containing 0.2 % Triton X-100, followed by incubation at room temperature for 3 to 4 hours. 20 $\mu$l of the resultant reaction mixture was placed in each well of a 96-well plate for use in fluorescence measurement (manufactured and sold by Greiner, Germany). To each well were added 160 $\mu$l of 1 mM EDTA, acetate buffer (pH 5.0) containing 0.2 % Triton X-100, and 20 $\mu$l of an acetonitrile solution containing 0.4 mM CPM (7-diethylamino-3-(4'-maleimidylphenyl)-4-methylcoumarin)(manufactured and sold by Molecular Probes, Inc., U.S.A.), followed by incubation at room temperature for 1 hour. The acetylcholinesterase activity of the resultant reaction mixture was measured by determining a fluorescence of 450 nm produced by the excitation with 365 nm light by means of PANDEX FCA (manufactured and sold by Baxter, U.S.A.).

When the measurement of an acetylcholinesterase activity was performed under serum-free conditions, an IMDM solution containing 1.0 % Nutridoma-SP (manufactured and sold by Boehringer-Mannhim GmbH, Germany) was used.

Reference Example 2 [the method for the measurement of the ploidy of the megakaryocyte]

The activity to promote the maturation of the megakaryocyte of the megakaryocyte potentiator of the present invention which was obtained by culturing a human cell, was directly confirmed as follows.

The above-obtained sample stained by the acetylcholinesterase staining solution, to be used in microscopy, was further stained in DAPI (4'-6-diamidino-2-phenylindole) staining solution for about 10 hours to thereby effect double-staining. About 200 megakaryocytes were arbitrarily selected from a colony thereof. The distribution of the ploidy among the cells was determined by means of BH2 type epi-fluorescent microscope and OSP-1 type fluorescent microscopic measuring device for DNA in a cell (both of which are manufactured and sold by Olympus Optical Co., Ltd., Japan). As the DAPI staining solution, use was made of 100 ml of a mixture prepared by mixing the following preserved solutions 1, 2 and 3 in a ratio of 0.5 ml: 98.5 ml: 1.0 ml, respectively.

Preserved solution 1:     a solution prepared by dissolving 10 mg of DAPI in 1000 ml of distilled water.
Preserved solution 2:     10 mM tris(hydroxymethyl) amionomethane buffer (pH 7.4) containing 0.1 M NaCl and 10 mM EDTA.
Preserved solution 3:     1 M 2-mercaptoethylamine hydrochloride solution

Example 1 [Production of megakaryocyte potentiator by the human cell culture method]

Commercially available normal diploid cells derived from the fetal human lung (available from Flow Laboratories, Inc., U.S.A), were planted in a glass bottle having a 100-liter volume at a density of $10^5$ cells/ml, together with Cytodex I (beads as a carrier for cell culture) (manufactured and sold by Pharmacia, Sweden) having a concentration of 2.5 mg/ml. 60 liters of a culture medium MEM containing 10 % fetal calf serum was added (as growth medium) to the cells, and the resultant mixture was subjected to suspension culturing at 37 °C in air containing 5 % $CO_2$ while stirring at 60 rpm. The cells were fully grown for 8 days before the carrier beads with the cells sticking thereto were washed with a physiological saline solution. The culture medium was replaced by 60 liters of a culture medium 199 which contained or did not contain 0.75

9

% of proteose peptone No. 3 having no serum, and the cells were further cultured while stirring at 60 rpm. Every three days during the culturing, the culture medium was replaced with a fresh medium and, at the same time, a conditioned medium containing the protein of the present invention was collected. The conditioned medium was concentrated into a 10-fold concentration. The obtained concentrate was tested for the megakaryocyte potentiator activity by the Meg-POT activity measurement using a soft agar culture medium as described in Reference Example 1(a). The results are shown in Table 1. For reference, cultures of several other types of cells were also measured with respect to the Meg-POT activity. The measurement revealed that fetal human lung cells have the ability to produce a megakaryocyte potentiator and that when the cells are cultured in the presence of a proteose peptone, a marked increase is observed in the production of the megakaryocyte potentiator.

(Table 1)

| Samples of conditioned medium | Number of colonies /$10^5$ cells |
|---|---|
| Conditioned medium of fetal human lung cells (+PP) | |
|     1st collection | 38 |
|     2nd collection | 35 |
|     5th collection | 26 |
|     10th collection | 33 |
| Conditioned medium of human embryonic lung cells (-PP) | |
|     1st collection | 6 |
|     5th collection | 7 |
| THP-1 (leukemia cells) | 2 |
| HepG2 (liver cancer cells) | 3 |
| T24   (bladder cancer cells) | 3 |
| MCF7  (breast cancer cells) | 0 |

[Note] 1)  The collection was conducted every three
              days of culturing.

       2)  PP: proteose peptone

The number of colonies for each sample is a value obtained by subtracting 3 from the actual number of colonies, which number 3 corresponds to the number of colonies occurring when only IL-3 is added to the assay system.

Example 2 [Purification of megakaryocyte potentiator: I]

About 1.1 liters of acetic acid was added to 170 liters of the supernatant of the culture of normal diploid cells derived from the fetal human lung obtained in Example 1, to adjust the pH to 4. The addition of acetic acid was followed by the removal of cell fragments and formed insoluble matter by filtration. The resultant mixture was adsorbed on a carboxymethyl sepharose (CM Sepharose, manufactured and sold by Pharmacia Fine Chemicals AB, Sweden) column (9 cm in diameter and 23.5 cm in height) fully equilibrated with 20 mM acetate buffer (pH 4.0) containing 0.2 M NaCl. After washing the column with 13.5 liters of the same equilibration buffer as mentioned above and with 6 liters of 20 mM acetate buffer (pH 4.0) containing 0.4 M NaCl, the column was eluted with 12 liters of 20 mM acetate buffer (pH 4.0) containing 0.75 M NaCl and 10 mM lysine monohydrochloride. About 5 liters of an eluate was obtained by the above operation. The eluate was designated as crude solution I having the megakaryocyte potentiator activity. The amount of proteose peptone, which had been contained in a large amount in the supernatant of the cell culture, was found to be decreased to 1 % or less.

The eluate (crude solution I) generally contains a large amount of tissue plasminogen activator (tPA). The tPA was specifically removed as follows: After 5 M sodium hydroxide solution was added to the eluate to adjust the pH to 7.0, the eluate was passed through an antibody column (9 cm in diameter and 29 cm in height) which contained a Sepharose gel having adhered thereto a monoclonal antibody against tPA (3 mg/ml gel) and had previously been fully equilibrated with 20 mM Tris•HCl buffer (pH 7.5) containing 0.5 M NaCl. By this operation, about 6 liters of a solution which was passed through the antibody column were obtained as crude solution II. By this column chromatography, the megakaryocyte potentiator was substantially quantitatively recovered. Further, the tPA contained in a large amount in the crude solution I, was also removed by the column chromatography.

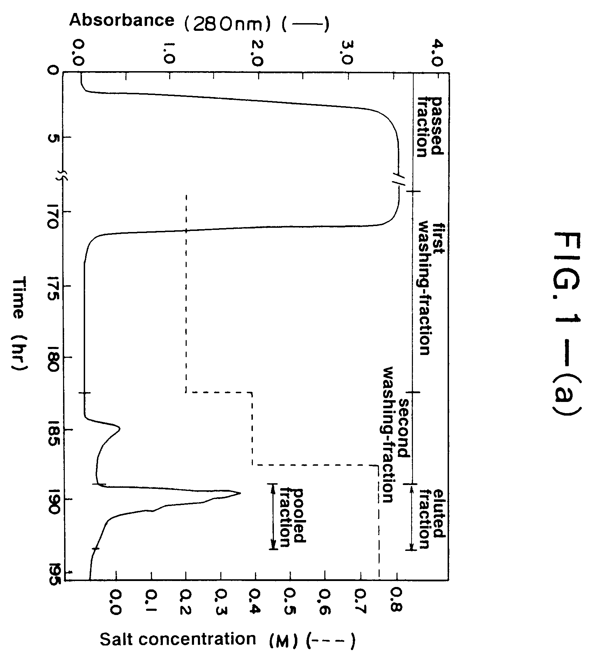

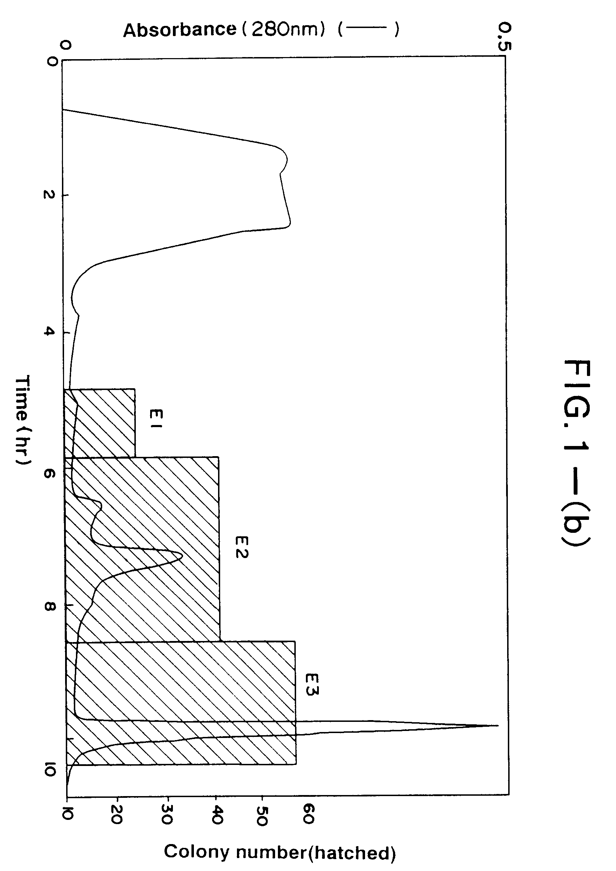

5.9 liters of the crude solution II were concentrated up to 300 ml by means of ultrafiltration module SIP-1013 (manufactured and sold by Asahi Kasei Kogyo K.K., Japan) and a 10-fold volume of 20 mM Tris•HCl buffer (pH 8.5) was added to the obtained concentrate. The resultant mixture was concentrated up to 300 ml again, so that the composition of the buffer was exchanged. The crude solution II was adsorbed onto a CM Sepharose Column (5 cm in diameter and 15 cm in height) fully equilibrated with 20 mM Tris•HCl buffer (pH 8.5). The column was washed with about 600 ml of the same buffer, and was eluted first with about 600 ml of the same buffer (E1) containing 0.05 M NaCl, then with about 600 ml of the same buffer (E2) containing 0.1 M NaCl, and finally with the same buffer (E3) containing 1 M NaCl, each at a flow rate of 200 ml/h. The eluted fractions were tested for megakaryocyte potentiator activity. The activity was observed mainly in the fractions obtained by E2 and E3. It was confirmed that the fraction E2 had an isoelectric point of 7 to 9 as measured by carrier-free isoelectric focusing, whereas the fraction of E3 contained a component having a more basic isoelectric point. The E2 fraction obtained in an amount of about 600 ml was designated as crude solution III. The megakaryocyte potentiator was recovered in this fraction at a recovery ratio of 20 to 40 %. Fig. 1-(a) shows an example of the results of the CM Sepharose column chromatography performed in the first stage of the purification and Fig. 1-(b) shows an example of the results of the CM Sepharose column chromatography performed in the third stage thereof.

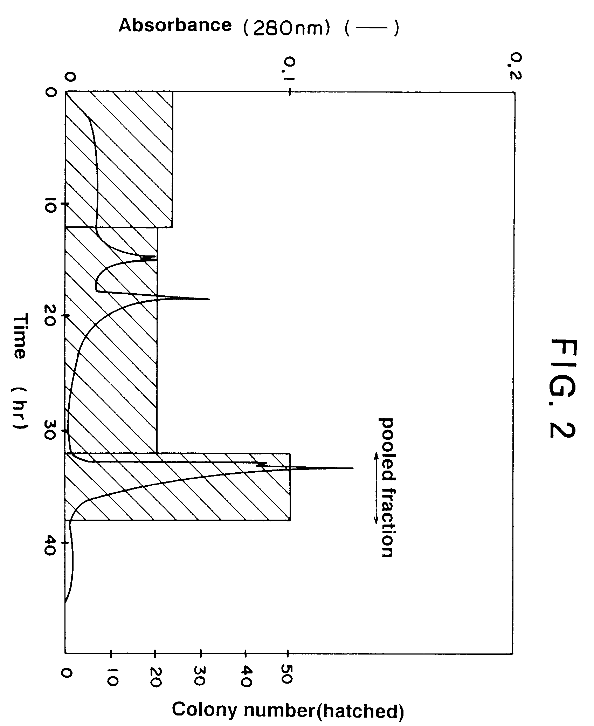

The above-mentioned crude solution III was fully dialyzed against 20 mM Tris•HCl buffer (pH 9.5) containing 10 mM NaCl. Then, the dialysate was adsorbed onto a Q Sepharose Column (5 cm x 5 cm) equilibrated with the same buffer. The column was washed with about 1 liter of the same buffer containing 50 mM NaCl, and was eluted with about 300 ml of 2 mM Tris•HCl buffer (pH 9.5) containing 0.2 M NaCl, at a flow rate of 50 ml/h. As a result, the megakaryocyte potentiator was recovered in the form of about 300 ml of the crude solution IV at an activity recovery ratio of 40 to 60 %. Fig. 2 shows an example of the results of the Q Sepharose column chromatography.

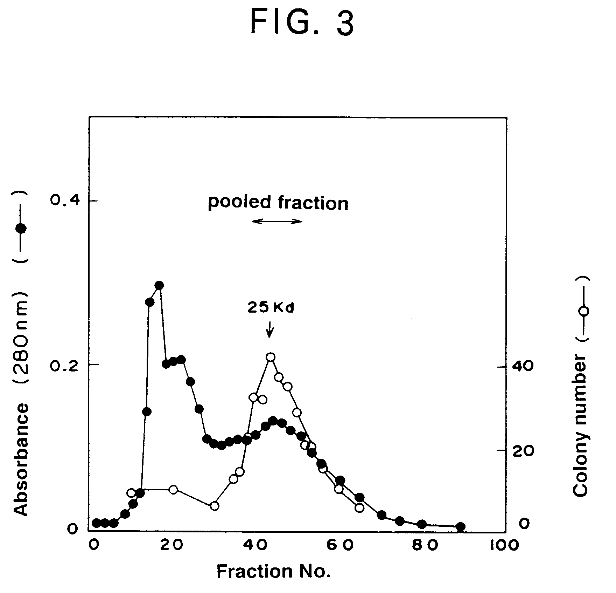

The above-obtained crude solution IV was concentrated to a 10-fold concentration by means of ultrafiltration hollow fibers and then subjected to gel filtration by means of a column (2.6 cm in diameter and 92 cm in height) which contained Sephacryl S-200 (manufactured and sold by Pharmacia Fine Chemicals AB, Sweden) equilibrated with PBS. The megakaryocyte potentiator (substantially purified product) was obtained at an activity recovery ratio of 40 to 60 % as a fraction in an amount of 50 ml having an elution peak at a molecular weight of about 25 kd. The total protein content was about 5 mg. The purification degree was about 1000-fold. Fig. 3 shows an example of the results of the Sephacryl S-200 column chromatography. The purification degree at each stage of the purification is shown in Table 2.

Table 2

| Sample | Volume (ml) | Protein recovery(%) | Activity recovery(%) | Purification degree(fold) |
|---|---|---|---|---|
| Culture Supernatant | 170000 | 100 | 100 | 1 |
| Crude solution I | 5120 | 0.40 | 68 | 170 |
| Crude solution II | 5900 | 0.28 | 61 | 218 |
| Crude solution III | 615 | 0.02 | 25 | 1250 |
| Crude solution IV | 303 | 0.005 | 12 | 2400 |
| Substantially purified product | 50 | 0.0007 | 7 | 10000 |

With respect to the crude solution IV and the substantially purified product, the protein concentration was determined by calculation, assuming that the ultraviolet-ray extinction coefficient at 280 nm

$$(A_{280}^{1\%}) = 10.$$

With respect to the other samples, the protein concentration was measured by the Lowry method.

Example 3 [Purification of megakaryocyte potentiator: II]

The megakaryocyte potentiator can be purified by the following method as well. 6 liters of a crude solution II was concentrated up to 300 ml by means of an ultrafiltration module SIP-1013 (manufactured and sold by Asahi Kasei Kogyo K.K., Japan) and a 10-fold volume of 20 mM phosphate buffer (pH 7.6) containing about 0.1 M NaCl was added to the concentrate. The resultant mixture was concentrated up to 300 ml again, so that the composition of the buffer was exchanged. The sample was adjusted to a pH of 5 and, immediately, it was adsorbed on a CM Sepharose column (5cm in diameter and 5cm in height) equilibrated with 20 mM citrate, phosphate buffer (pH 5.0) containing 0.05 M NaCl. After the column was washed with 400 ml of the same buffer, elution was conducted with a linear concentration gradient of NaCl between 0.05 and 0.5 M in the same buffer, at a flow rate of 60 ml/h. A megakaryocyte potentiator activity was recovered at a recovery ratio of 30 to 50%, as a fraction which exhibited a peak when the NaCl concentration was between 0.05 and 0.5 M. This fraction obtained in an amount of about 100 ml was designated as crude solution III'.

100 ml of the crude solution III' was concentrated up to a 5-fold concentration by means of the above-mentioned hollow fiber ultrafiltration filter and the concentrated solution was subjected to gel filtration using a Sephacryl S-200 column (which Sephacryl is manufactured and sold by Pharmacia Fine Chemicals AB, Sweden)(2.6 cm in diameter and 92 cm in height) fully equilibrated with PBS. A megakaryocyte potentiator activity was recovered at a recovery ratio of 40-60 % as a fraction which exhibited an elution peak at a molecular weight of about 25 kd. This fraction obtained in an amount of about 50 ml was designated as crude solution IV'.

The crude solution IV' was adsorbed onto a 10 ml of Orange Sepharose (manufactured and sold by Amicon, U.S.A.) and a fraction eluted at an NaCl concentration of 1M was taken, to thereby obtain about 8 mg of a substantially purified product of the megakaryocyte potentiator. The purification degree was 7000-fold.

The purification degree attained in each stage of the purification is shown in Table 3.

With respect to the crude solution IV' and the substantially purified product, the protein concentration was determined by calculation on the assumption that the ultraviolet extinction coefficient at 280 nm

$$(A_{280}^{1\%}) = 10.$$

In the case of the other samples, the protein concentration was measured by the Lowry method.

Table 3

| | Sample | Volume (ml) | Protein recovery (%) | Activity recovery (%) | Purification Degree (fold) |
|---|---|---|---|---|---|
| | Culture supernatant | 170000 | 100 | 100 | 1 |
| | Crude solution I | 5050 | 0.38 | 64 | 168 |
| | Crude solution II | 6120 | 0.30 | 58 | 193 |
| | Crude solution III' | 97 | 0.05 | 21 | 420 |
| | Crude solution IV' | 46 | 0.005 | 10 | 2000 |
| | Substantially purified product | 52 | 0.001 | 7 | 7000 |

Example 4 [Properties of megakaryocyte potentiator]

The protein of the present invention was tested for the following characteristics.

Molecular weight

The substantially purified products obtained in Examples 2 and 3 were individually gel-filtered with PBS (at a flow rate of 27.6 ml/h) using a column of Sephacryl S-200HR (manufactured and sold by Pharmacia Fine Chemicals AB, Sweden) (2.6 cm in diameter and 94 cm in height) fully equilibrated with PBS. The eluate were fractionated into fractions, each having a volume of 4.6 ml. The molecular weight of the megakaryocyte potentiator of each fraction was measured by a method in which the elution positions of proteins of a low molecular weight marker protein kit for gel filtration (BSA: 67 kd, Ovalbumin: 43 kd, chymotrypsinogen: 25 kd, and ribonuclease A: 14 kd; manufactured and sold by Pharmacia Fine Chemicals AB, Sweden) and the elution position of Blue Dextran 2000 were confirmed, and the elution position of the fraction was compared with the thus confirmed elution positions. The protein of the present invention was eluted with a peak at about 25 kd.

Isoelectric point

1 ml of each of the substantially purified products obtained in Example 2 and 3 was dialyzed overnight against 2 mM phosphate buffer. After replacement of the solvent, glycerol density gradient isoelectric focusing was conducted with respect to the purified products under the following conditions. The measurement results showed that the protein of the present invention has an isoelectric point in the range of pH = 7 to 9.

Column for isoelectric focusing: 110 ml volume (manufactured and sold by Kato Shoichi's Store, Japan)

Carrier ampholite: 1 % Ampholine 3.5 - 10 (manufactured and sold by Pharmacia LKB Biotechnology, Sweden)

Glycerol density gradient: 0 - 60 %

Electric Power: 3 W

Operation Time: 40 hours.

Thermal stability and trypsin treatment stability

The substantially purified products obtained in Examples 2 and 3 were individually subjected to heat treatment in PBS at 100°C for 10 minutes to evaluate their stability based on the residual megakaryocyte potentiator activity. The activity of the protein of the present invention was reduced to 5 % or less, indicating that it is unstable. This protein also exhibited a high sensitivity to treatment with 0.125 mg/ml of trypsin at 37°C for one hour.

Antigenicity

By the use of the substantially purified products obtained in Examples 2 and 3, the protein of the present invention was tested as to whether or not it would immunologically react to known cytokines. That is, a neutralization testing of the megakaryocyte potentiator activity of the present protein was carried out by exposing the protein to antihuman erythropoietin antibody, antihuman IL-1α antibody, antihuman IL-1β

EP 0 517 925 A1

antibody, antihuman IL-6 antibody, and antihuman IL-7 antibody (all of these antibodies are manufactured and sold by Genzyme Corporation, USA). The results showed that even after the protein of the present invention was exposed to these antibodies, substantially no lowering was observed in the activity of the protein, indicating that the protein of the present invention does not react to these antibodies. Further, the protein of the present invention was not adsorbed on an antiIL-1 antibody column and an antiIL-6 antibody column (manufactured and sold by Endogen Inc., U.S.A). These results indicate that the megakaryocyte potentiator of the present invention is immunologically distinguished from these known cytokines.

Example 5 [Megakarytocyte potentiator activity]

Using the substantially purified products obtained in Examples 2 and 3, the megakaryocyte potentiator activity of the protein of the present invention was measured by the soft agar culture method. For comparison, the Meg-POT activities of IL-6 and IL-11 were also measured. The rhIL-6 derived from CHO cells employed was a commercially available product (manufactured and sold by Genzyme Corporation, U.S.A). Further, the employed rhIL-11 derived from COS 1 cells and that from CHO cells were prepared by the following procedures.

Preparation of human embryonic lung (HEL) cell cDNA library

According to the modified guanidine isothiocyanate method, about 200 $\mu$g of total RNA was extracted from about 2 x $10^8$ human embryonic lung cells. In the extraction, a total RNA isolation kit (manufactured and sold by Invitrogen Corporation, USA) was used. Subsequently, about 1.6 $\mu$g of RNAs having poly A attached thereto were isolated from the total RNA by use of Oligodex. The synthesis of a cDNA was performed by the Okayama-Berg method, as follows. Using 3'oligo(dT)-tailed pcDV-1 (manufactured and sold by Pharmacia Fine Chemicals AB, Sweden) as a vector primer, the cDNA synthesis from poly A-tailed RNAs was carried out using a cDNA synthesis kit (manufactured and sold by Boehringer-Mannhim GmbH, Germany) according to the manual accompanying the kit. After completion of the synthesis reaction, phenol extraction and ethanol precipitation were performed, and then dC tail was attached to the reaction product by use of a tailing kit (manufactured and sold by Boehringer-Mannhim GmbH, Germany). After further phenol extraction and ethanol precipitation, the reaction product was digested with restriction enzyme HindIII, and then it was subjected to phenol/chloroform extraction and ethanol precipitation. Next, the reaction product was heated together with 0.5 ng of 3'oligo(dG)-tailed pL-1 HindIII linker (manufactured and sold by Pharmacia Fine Chemicals AB, Sweden) at 65 °C for 5 minutes, and then at 42 °C for 60 minutes. After heating, it was cooled to room temperature. Subsequently, the reaction product was cyclized by E. coli DNA ligase. Then, RNA chain was converted to DNA chain by use of RNaseH, DNA polymerase I and DNA ligase. The transformation of E. coli K12 MC1061 (obtained from Beckman City of Hope Medical Institute) using the thus synthesized cDNA was conducted according to the conventional method.

Screening of IL-11 from HEL cell cDNA library

First, an oligonucleotide having the following sequence: 5'-CCGAGGGTCTGGGGAAACTC-3' was synthesized by means of a DNA synthesizer (DNA Synthesizer Model 980-A manufactured and sold by Applied Biosystems Co., Ltd., U.S.A.) according to the conventional method. Next, the 5' terminal of the above-mentioned oligonucleotide was phosphorylated by T4 polynucleotide kinase, to thereby obtain a synthetic DNA probe for IL-11. A colony hybridization of the cDNA library prepared as mentioned above was conducted, using the above-obtained probe, in accordance with a laboratory manual (Maniatis et al., Molecular Cloning 2nd. Edition 1.85, 1989, Cold Spring Harbor Laboratory). As a result, by a screening of about 70,000 transformants, several clones which were able to react to the probe were obtained. A plasmid DNA (hereinafter referred to as pcDIL-11-12) was prepared from one of the obtained clones according to the laboratory manual.

Determination of nucleotide sequence

Using a restriction enzyme, portions of the obtained plasmid which were considered to cover the coding region of IL-11 gene, were subcloned into the multi-cloning sites of M13mp18 and M13mp19. Subsequently, the nucleotide sequence was determined by use of a fluorescent DNA sequencer (DNA Sequencer 373A manufactured and sold by Applied Biosystems Co., Ltd., U.S.A.) and a fluorescent primer cycle sequencing kit (manufactured and sold by Applied Biosystems Co., Ltd., Catalog No. 401119) according to the protocol

14

accompanying the DNA sequencer. When the obtained nucleotide sequence was compared with that of a human IL-11 cDNA, the primary structure of amino acids as deduced from the nucleotide sequence, was completely in agreement with that of the human IL-11. Therefore, it was considered that the gene contained in the above-obtained plasmid was surely the human IL-11 gene.

Expression of plasmid pcDIL-11-12 in COS-1 cells

Transfection of the plasmid to a COS cell was performed according to the conventional method as follows. COS-1 cell (ATCC CRL 1650) was cultured to about 50 % of confluence on Dulbecco's modified minimum essential medium (hereinafter referred to as DMEM; manufactured and sold by Flow Laboratories Inc., U.S.A.) supplemented with 10 % (v/v) fetal calf serum (hereinafter referred to as FCS; manufactured and sold by GIBCO, U.S.A.) in a dish for tissue culture in an incubator containing 5 % $CO_2$ at 37 ° C. Immediately before the transfection, the cells were washed with PBS (-) (manufactured and sold by Nissui Pharmaceutical Co., Japan) prepared in accordance with the accompanying manual. The culture medium was replaced with 4 ml of DMEM supplemented with 10 % (v/v) Nuserum I (manufactured and sold by Collaborative Research, Inc., U.S.A.). Separately, a plasmid pcDIL-11-12 DNA (10 $\mu$g) dissolved in 60 $\mu$l of TBS was dropwise added in 120 $\mu$l of a 10 mg/ml DEAE-dextran solution to prepare a DNA/DEAE-dextran solution. Next, this DNA/DEAE-dextran solution was dropwise added to the above-mentioned dish so that the solution uniformly covered the cells. Then the cells were cultured in an incubator containing 5 % $CO_2$ at 37 ° C for 4 hours. After a culture supernatant was removed by suction, 5 ml of PBS (-) supplemented with 10 % (v/v) of DMSO (Manufactured and sold by Merck & Co., Inc., U.S.A.) was added to the cells and the mixture was allowed to stand at room temperature for one minute, followed by removal of the PBS by suction. The cells were washed with 5 ml of PBS (-) and then, 7 ml of DMEM supplemented with 100 $\mu$M Chloroquine (manufactured and sold by Wako Pure Chemical Industries, Ltd., Japan) and 2 % (v/v) of FCS, was added to the cells, and the cells were cultured in an incubator containing 5 % $CO_2$ at 37 ° C for three hours. After that period, the culture supernatant was removed and the cells were washed with 10 ml of PBS(-). Then, DMEM containing, added thereto, 10 % FCS was added to the cells and the cells were cultured in an incubator containing 5 % $CO_2$ at 37 ° C for two days. Then, the medium was replaced with serum-free DMEM and, thereafter, the supernatant of the culture was collected every two days.

Introduction of plasmid pcDIL-11-12 to Chinese hamster ovary cell (CHO) and expression thereof

CHO-dhfr⁻cell strain was obtained from Dr. L. Chasin and Dr. G.U. Chasin of Columbia University. First, 5 x 10⁵ of CHO-dhfr⁻cells were cultured at 37 ° C for a day in a dish for tissue culture, by use of a growth culture medium [Ham's F-12 medium (manufactured and sold by Flow Laboratories, Inc., U.S.A.) supplemented with 10 % of FCS].

The obtained plasmid pcDIL-11-12 and plasmid pSV2-dhfr (ATCC No. 37146) were introduced into the CHO-dhfr⁻cell strain by using the calcium phosphate method in accordance with the F.L. Graham et al.'s method [F.L. Graham, Virology, 52, 456 (1973)]. The cells were cultured in a growth culture medium for three days and then, the growth culture medium was replaced with a selective medium (DMEM)-(manufactured and sold by Flow Laboratories, Inc. U.S.A.) containing proline added thereto at a concentration of 150 $\mu$g/ml and a dialyzed fetal calf serum (manufactured and sold by GIBCO, U.S.A.) added thereto at a concentration of 10 %. Thereafter, the culture medium was replaced with a fresh one every three days. About two weeks later, transformed cell colonies appeared. Several colonies were picked up and subjected to expansion culturing. The supernatant of the culture was tested for the megakaryocyte potentiator activity in accordance with Reference Example 1(a). As a result, some clones having megakaryocyte potentiator activity were detected. The obtained colonies having the activity were further cultured for about two weeks in a selective medium containing 20 nM methotrexate (MTX)(manufactured and sold by Wako Pure Chemical Industries, Ltd., Japan) to obtain MTX-resistant colonies. Several colonies were picked up and subjected to expansion culturing. Subsequently, in accordance with Reference Example 1(a), the supernatant of the culture was measured for the megakaryocyte potentiator activity. Some clones having an enhanced activity were detected. Further, the substantially same procedure was repeated except that the MTX concentration was increased to 200 nM, to thereby obtain MTX-resistant strains. One of these strains was used as an IL-11-producing strain in the subsequent experiment. The strain was cultured in a selective culture medium until it became confluent. Subsequently, the strain was cultured in a selective culture medium which was free from fetal calf serum, and the resultant culture supernatant was collected.

Measurement of Megakaryocyte Potentiator Activity

The measurement of the megakaryocyte potentiator activity was made by the soft agar culture method. The results are shown in Table 4. With respect to the Meg-POT activity attained by the co-use of IL-6 (manufactured and sold by Genzyme Corporation, U.S.A.) with IL-3, no clear activity was exhibited at concentrations of 1 ng/ml and 50 ng/ml. Even at a concentration of 200 ng/ml, the Meg-POT activity attained by the co-use of IL-6 was only about three times the activity of IL-3 alone. By contrast, at a concentration of 1 ng/ml, the substantially purified protein of the present invention, when used in combination with IL-3, exhibited Meg-POT activity six times that of IL-3 alone. At a concentration of 10 ng/ml, the protein of the present invention formed as much as about 50 colonies which were in a state of saturation. Also, the Meg-POT activity attained by the co-use of IL-11 with IL-3 was measured with respect to the supernatant of each of cultures of COS1 and CHO cells in which IL-11 was expressed. The maximum number of colonies obtained by the co-use of IL-11 was somewhat larger than that obtained by the co-use of IL-6, while the maximum number of colonies obtained by the co-use of IL-11 was only about half that obtained by the co-use of the protein of the present invention, indicating that the Meg-POT activity of IL-11 was largely lower than that of the protein of the present invention. The protein of the present invention alone, did not promote the proliferation of a megakaryocyte. Further, the activities of human granulocyte colony stimulating factor (G-CSF) and human macrophage stimulating factor (M-CSF) were also measured. Each of these factors neither exhibited substantial Meg-POT activity when it was used in combination with IL-3, nor Meg-CSF activity even when it was used alone.

Further, the ploidy attained with respect to the use of IL-3 alone or IL-3 plus the protein of the present invention (10 ng/ml) was determined by the measurement of the content of the megakaryocyte chromosomal DNA. As a result, it was found that when only IL-3 was used, the number of cells which had a ploidy of 4n to 8n was larger than the number of the other cells, whereas when the protein of the present invention was used, the proportion of cells having a ploidy of 16n or more reached 60 % or more, indicating that the protein of the present invention has a high Meg-POT activity.

Table 4

| | Type of sample | | Colony Number |
|---|---|---|---|
| IL-3 | only (10 ng/ml) | | 3 |
| IL-3 | + | the protein of the present invention (1 ng/ml) | 18 |
| IL-3 | + | the protein of the present invention (10 ng/ml) | 48 |
| IL-3 | + | the protein of the present invention (100 ng/ml) | 47 |
| | | the protein of the present invention (10 ng/ml) | 0 |
| IL-3 | + | IL-6 (1 ng/ml) | 3 |
| IL-3 | + | IL-6 (50 ng/ml) | 5 |
| IL-3 | + | IL-6 (200 ng/ml) | 9 |
| IL-3 | + | IL-11 (COS1)(1 $\mu$l/ml) | 7 |
| IL-3 | + | IL-11 (COS1)(50 $\mu$l/ml) | 24 |
| IL-3 | + | IL-11 (COS1)(100 $\mu$l/ml) | 20 |
| IL-3 | + | IL-11 (CHO)(1 $\mu$l/ml) | 8 |
| IL-3 | + | IL-11 (CHO)(50 $\mu$l/ml) | 25 |
| L-3 | + | IL-11 (CHO)(100 $\mu$l/ml) | 26 |
| IL-3 | + | GCSF (50 ng/ml) | 2 |
| | | GCSF (50 ng/ml) | 0 |
| IL-3 | + | MCSF (10 ng/ml) | 3 |
| | | GCSF (10 ng/ml) | 0 |

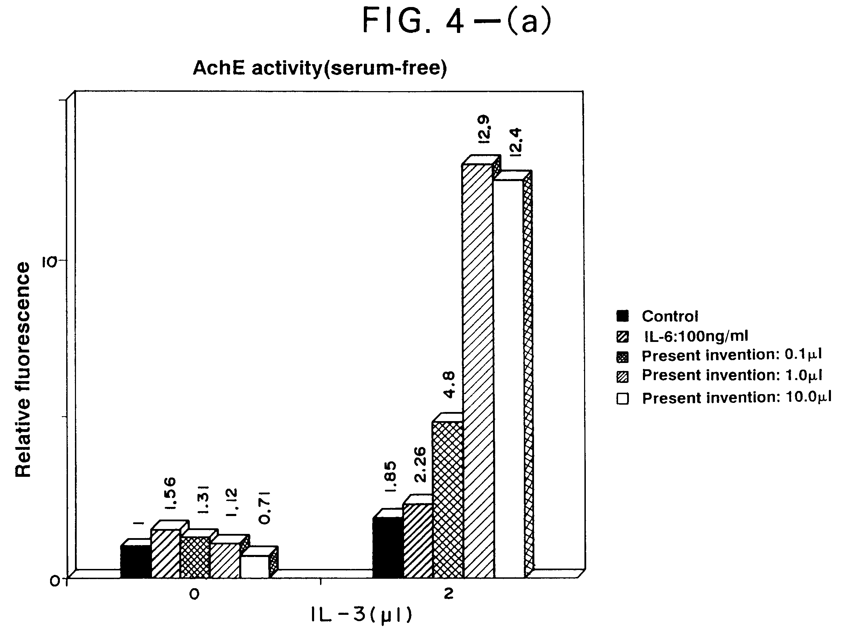

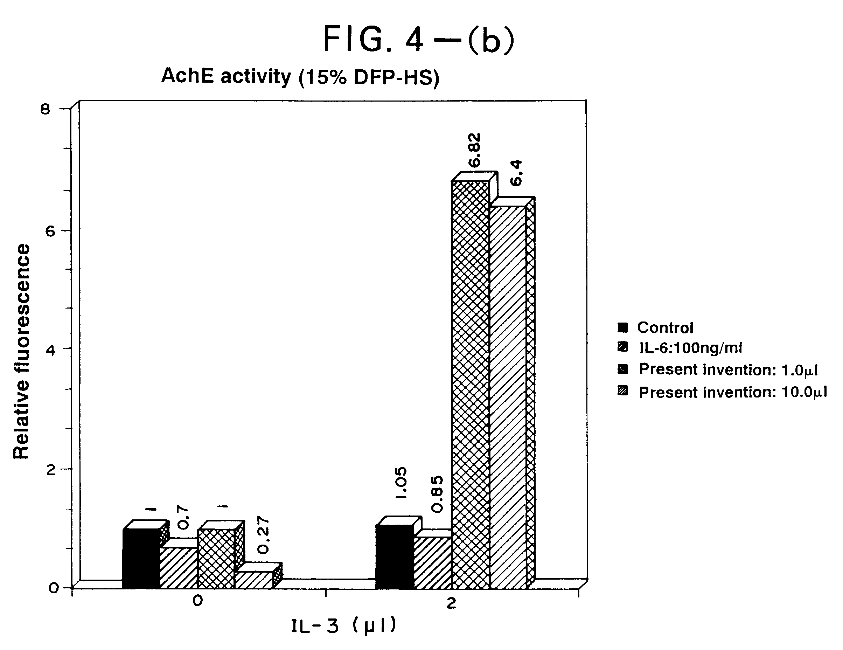

Further, the Meg-POT activity of the protein of the present invention was evaluated by measuring acetylcholinesterase activity (AchE activity) using liquid culturing. In order to make a comparison, IL-6 also was evaluated for the Meg-POT activity. In this method, 2 $\mu$l of IL-3 as Meg-CSF was added to IL-6, and only IL-3 was used as a control. The results are shown in Fig. 4-(a) and Fig. 4-(b). Bars on the left-hand portion of each of Figs. 4-(a) and 4-(b) represent data obtained under conditions where there was no addition of IL-3. The relative fluorescence is shown as a value relative to value 1 obtained with respect to the control, namely, a sample containing none of the IL-3, IL-6 and the protein of the present invention. Fig.

4-(a) shows the results of the culturing under serum-free conditions. Fig. 4-(b) shows the results of the culturing in the presence of 15 % horse serum (HS) treated with DFP. Also in this measuring method, it was found that the protein of the present invention exhibits a strong megakaryocyte potentiator activity.

Example 6 [Thrombopoietin action of the megakaryocyte potentiator]

The substantially purified protein of the present invention was administered to the abdominal cavities of mice (C57BL male, 7 weeks old, 5 mice per group) continuously for 5 days. 3 hours after the final administration, blood was taken from them, and the numbers of platelets and red blood cells were counted. As shown in Table 5, the protein of the present invention was found to have a thrombopoietin activity to thereby significantly increase the number of platelets with of peril rate (P) of 1 % or less. The number of red blood cells did not show an increase. Referring to Table 5, 1 $\mu$g per dose and 0.2 $\mu$g per dose of the protein of the present invention dissolved in PBS containing 150 $\mu$g/ml of bovine serum alubmin (BSA), were administered to the mice of Group 1 and Group 2, respectively. Only BSA was administered to Group 3 as a control group.

(Table 5)

| | Number of platelets (x$10^4$/$\mu$l) | Number of red blood cells (x$10^4$/$\mu$l) |
|---|---|---|
| Group 1 (1 $\mu$g of the protein of the present invention) | 146.2 ± 9.9 | 1135 ± 44 |
| Group 2 (0.2 $\mu$g of the protein of the present invention) | P<0.01 122.5 ± 10.5 | 1074 ± 32 |
| Group 3 (150 $\mu$g/ml BSA) | 98.4 ± 12.1 | 1030 ± 120 |

[Note]  BSA: bovine serum albumin

Example 7

Examples of formulations for pharmaceutical compositions containing the megakaryocyte potentiator of the present invention as an active ingredient, and a method for preparing the pharmaceutical compositions are described below. The formulation examples should not be construed to be limiting the scope of the present invention.

(Formulation Example 1)

| | |
|---|---|
| Substantially purified megakaryocyte potentiator of the present invention | 1 mg |
| Purified gelatin | 20 mg |
| Mannitol | 100 mg |
| Sodium chloride | 7.8 mg |
| Sodium phosphate | 15.4 mg |

The above-mentioned ingredients were dissolved in 2 ml of distilled water for injection, and the resultant solution was placed into a sterile vial. The solution was subjected to a primary drying at -35°C under a vacuum degree of 0.075 Torr for 35 hours and then, subjected to a secondary drying at 30°C under a vacuum degree of 0.03 Torr for 5 hours, to thereby prepare a vial for injection. The thus obtained composition is to be dissolved in 500 ml of a physiological saline solution or a glucose injection just before use for an intravenous drip infusion.

(Formulation Example 2)

| Substantially purified megakaryocyte potentiator of the present invention | 10 $\mu$g |
|---|---|
| Albumin | 5 mg |
| Mannitol | 25 mg |
| Sodium chloride | 1.95 mg |
| Sodium phosphate | 3.85 mg |

Using the above ingredients, a vial for injection was prepared in substantially the same manner as in Formulation Example 1 above.

Brief Description of the Drawings

Fig. 1 shows a chromatogram in each stage of the purification in Example 2. Fig. 4-(a) shows the results of the CM Sepharose column chromatography in the first stage of the purification, and Fig. 4-(b) shows the results of the CM Sepharose column chromatography in the third stage of the purification.

Fig. 2 shows the results of the Q Sepharose column chromatography in the fourth stage of the purification in Example 2.

Fig. 3 shows the results of the gel filtration in Example 2.

Fig. 4 shows the results of the measurement of the megakaryocyte potentiator activities with respect to the megakaryocyte potentiator of the present invention and IL-6, which measurement was done by measuring acetylcholinestrase activity (AchE activity) using liquid culturing. Fig. 4-(a) shows the results of the culturing under serum-free conditions, and Fig. 4-(b) shows the results of the culturing in the presence of 15 % horse serum (HS) treated with DFP.

Industrial Applicability

The megakaryocyte potentiator protein of the present invention has not only the activity to promote the proliferation and differentiation of a megakaryocyte but also the activity to increase the number of platelets in peripheral blood. The activity exhibited by the megakaryocyte potentiator of the present invention is high, as compared to the conventional factors having similar activities to the megakaryocyte potentiator activity. Therefore, the megakaryocyte potentiator protein of the present invention can be used in a single form, or in the form of a pharmaceutical composition containing the protein as an active ingredient, for the purpose of preventing and treating thrombocytopenia and the like.

**Claims**

1. A substantially pure megakaryocyte potentiator protein having the activity to promote the proliferation and differentiation of a megakaryocyte and which has the following characteristics:
   (a) a molecular weight of 25,000 ± 8,000 as measured by a gel filtration method;
   (b) an isoelectric point of 8 ± 1 as measured by an isoelectric focusing method;
   (c) the activity to promote the proliferation and differentiation of a megakaryocyte is substantially not lowered when said protein is tested after exposure to each of the antibodies against human erythropoietin, human interleukin 1$\alpha$, human interleukin 1$\beta$, human interleukin 6 and human interleukin 7; and
   (d) no megakaryocyte colony stimulating factor activity.

2. The megakaryocyte potentiator protein according to claim 1, which is derived from human cells.

3. The megakaryocyte potentiator protein according to claim 2, wherein said human cells are normal diploid cells.

4. The megakaryocyte potentiator protein according to claim 3, wherein said normal diploid cells are

18

derived from lung.

5. A method for producing a megakaryocyte potentiator protein, which comprises culturing animal cells in a culture medium to thereby produce a megakaryocyte potentiator protein in a conditioned medium, collecting a supernatant from said conditioned medium, and isolating and purifying said megakaryocyte potentiator protein from said supernatant, thereby obtaining a megakaryocyte potentiator protein having the activity to promote the proliferation and differentiation of a megakaryocyte,

said megakaryocyte potentiator protein having the following characteristics:

(a) a molecular weight of 25,000 ± 8,000 as measured by a gel filtration method;

(b) an isoelectric point of 8 ± 1 as measured by an isoelectric focusing method;

(c) the activity to promote the proliferation and differentiation of a megakaryocyte is substantially not lowered when said protein is tested after exposure to each of the antibodies against human erythropoietin, human interleukin 1$\alpha$, human interleukin 1$\beta$, human interleukin 6 and human interleukin 7; and

(d) no megakaryocyte colony stimulating factor activity.

6. The method according to claim 5, wherein said animal cells are human cells.

7. The method according to claim 6, wherein said human cells are normal diploid cells.

8. The method according to claim 7, wherein said normal diploid cells are derived from lung.

9. The method according to claim 5, wherein said culture medium for culturing the animal cells contains an agent capable of promoting the production of a megakaryocyte potentiator protein.

10. The method according to claim 9, wherein said agent capable of promoting the production of a megakaryocyte potentiator protein is a peptone derived from animal meat.

11. The method according to claim 10, wherein said cells are derived from human lung.

12. A method for producing a megakaryocyte potentiator protein, which comprises:

(a) extracting RNA from animal cells;

(b) obtaining poly A$^+$ RNA from said RNA;

(c) preparing a cDNA library from an expression vector and said poly A$^+$ RNA obtained in step (b);

(d) preparing plasmid DNA from said cDNA library using first host cells;

(e) transfecting second host cells with said plasmid DNA;

(f) culturing said second transfected host cells, or cells which are obtained by screening said second transfected host cells using as a criterion the activity to promote the proliferation and differentiation of a megakaryocyte, to thereby express a megakaryocyte potentiator protein; and

(g) collecting and purifying said expressed megakaryocyte potentiator protein.

13. A pharmaceutical composition comprising a therapeutically effective amount of the megakaryocyte potentiator protein of claim 1 and at least one pharmaceutically acceptable carrier, diluent or excipient.

14. The pharmaceutical composition according to claim 13, which further comprises at least one factor selected from the group consisting of IL-1, IL-2, IL-3, IL-4, IL-5, IL-6, IL-7, IL-8, IL-9, IL-11, GM-CSF, G-CSF, M-CSF, SCF, IFNs, LIF, TNF and EPO.

EP 0 517 925 A1

# FIG. 1 —(a)

FIG.1—(b)

FIG. 2

# FIG. 3

# FIG. 4 — (a)

**AchE activity(serum-free)**

Legend:
- ■ Control
- ▨ IL-6:100ng/ml
- ▦ Present invention: 0.1µl
- ▧ Present invention: 1.0µl
- □ Present invention: 10.0µl

Y-axis: Relative fluorescence

X-axis: IL-3(µl)

Values at IL-3 = 0: 1, 1.56, 1.31, 1.12, 0.71
Values at IL-3 = 2: 1.85, 2.26, 4.8, 12.9, 12.4

24

# FIG. 4 — (b)

## AchE activity (15% DFP-HS)

Legend:
- ■ Control
- ▨ IL-6:100ng/ml
- ▦ Present invention: 1.0μl
- ▧ Present invention: 10.0μl

25

# INTERNATIONAL SEARCH REPORT

International Application No PCT/JP91/01803

**I. CLASSIFICATION OF SUBJECT MATTER** (if several classification symbols apply, indicate all) [6]

According to International Patent Classification (IPC) or to both National Classification and IPC

Int. Cl[5]   C07K15/04, C12P21/00, A61K37/02//C12N15/19
            (C12P21/00, C12R1:91)

**II. FIELDS SEARCHED**

| Minimum Documentation Searched [7] | |
|---|---|
| Classification System | Classification Symbols |
| IPC | C07K15/04, C12P21/00-21/02, A61K37/02, C12N15/19 |

Documentation Searched other than Minimum Documentation
to the Extent that such Documents are Included in the Fields Searched [8]

Biological Abstracts Data Base (BIOSIS)

**III. DOCUMENTS CONSIDERED TO BE RELEVANT** [9]

| Category [*] | Citation of Document, [11] with indication, where appropriate, of the relevant passages [12] | Relevant to Claim No. [13] |
|---|---|---|
| A | JP, A, 63-239298 (Massachusetts Institute of Technology), October 5, 1988 (05. 10. 88), & EP, A2, 260918 & US, A, 4894440 | 1-14 |
| A | Leukemia Research, Vol. 10, No. 4, (1986), O. S. Huat et al. "Biochemical characterization of an in-vitro murine megakaryocyte growth activity: megakaryocyte potentiator" p. 403-412 | 1-14 |
| A | Journal of Biological Chemistry, Vol. 262, No. 7, (1987), G. Tayrien et al. "Purification and properties of a Megakaryocyte Stimulatory Factor Present both in the serum-free conditioned and medium of human embryonic kidney cells and in Thrombocytopenic plasma" p. 3262-3268 | 1-14 |

[*] Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| March 24, 1992 (24. 03. 92) | April 21, 1992 (21. 04. 92) |
| International Searching Authority | Signature of Authorized Officer |
| Japanese Patent Office | |

Form PCT/ISA/210 (second sheet) (January 1985)